# EUROPEAN PATENT SPECIFICATION

(11) **EP 2 961 767 B1**
(45) Date of publication and mention of the grant of the patent: **26.09.2018**
(21) Application number: 14709396.7
(22) Date of filing: 03.03.2014
(51) Int. Cl.: C07K 7/64, A61K 38/04

(54) **NON-IMMUNOSUPPRESSIVE CYCLOSPORIN DERIVATIVES AS ANTIVIRAL AGENTS**
NICHT-IMMUNSUPPRESSIVE CYCLOSPORINDERIVATE ALS VIRUZIDE
DÉRIVÉS DE CYCLOSPORINE NON IMMUNODÉPRESSEURS EN TANT QU'AGENTS ANTIVIRAUX

(30) Priority: 01.03.2013 GB 201303681; 01.03.2013 GB 201303685
(43) Date of publication of application: 06.01.2016
(73) Proprietor: UCL Business PLC, London, W1T 4TP (GB)
(72) Inventor: TOWERS, Greg, London E8 1PD (GB); SELWOOD, David, Welwyn Garden City Hertfordshire AL8 6EY (GB); RASAIYAAH, Jane, London N19 5PW (GB)
(74) Representative: J A Kemp
(86) International application number: PCT/GB2014/050612
(87) International publication number: WO 2014/132084

(56) References cited:
- H DUBE ET AL.: "A mitochondrial-targeted cyclosporin A with high binding affinity for cyclophilin D yelds improved protection of cardiomyocytes", BIOCHEMICAL JOURNAL, vol. 441, 2012, pages 901-907, XP002725732, Portland Press Ltd. ISSN: 0264-6021
- J-C CARRY ET AL.: "semisynthetic Di- and Tri-functionalized non-immunosuppressive cyclosporin A derivatives as potential anti-HIV 1 drugs", SYNLETT, vol. 2, 2 February 2004 (2004-02-02), pages 316-319, XP002578412, GEORG THIEME VERLAG ISSN: 0936-5214
- J RASAIYAAH ET AL.: "HIV-1 evades innate immune recognition through specific cofactor recruitment", NATURE, vol. 503, 21 November 2013 (2013-11-21), pages 402-405, XP002725733, Nature Publishing Group ISSN: 0028-0836

## Description

### BACKGROUND

Human Immunodeficiency Virus 1 (HIV-1) is thought to have infected up to 60 million people since its discovery over 20 years ago. Of those infected, more than 20 million have died, with the vast majority of individuals affected being from developing countries. There exists a need for more effective treatments for HIV-1 infections.

HIV-1 is able to replicate in primary human macrophages without stimulating a innate immune response, despite reverse transcription of genomic RNA into double stranded DNA, an activity that might be expected to trigger innate pattern recognition receptors (PRRs). HIV-1 replication is thus cloaked in human macrophages, so that it is invisible to the cell autonomous innate immune system. Very little is known of the mechanism behind the cloaking of HIV-1 replication. However, interference with this cloaking mechanism should result in activation of PRRs and a corresponding innate immune response via interferon secretion. Disruption of the HIV-1 cloaking mechanism could therefore provide an effective treatment for patients infected with HIV-1, by activation of their innate immune response.

### SUMMARY OF THE INVENTION

The present inventors have surprisingly found that certain derivatives of cyclosporin are able to disrupt the HIV-1 cloaking mechanism, which means that PRRs are activated and interferons are secreted. The innate immune response is thus activated. It has also surprisingly been found that a similar anti-viral effect is observed, via activation of PRRs, with other viruses, such as human cytomegalovirus (hCMV).

Furthermore, it has surprisingly been found that these derivatives of cyclosporin are non-immunosuppressive, unlike cyclosporin itself. The compounds of the invention are therefore suitable for administration to patients infected with viruses, such as HIV-1, who will often have compromised immunity.

The combined properties of high anti-viral activity and low immunosuppressivity associated with the cyclosporin derivatives of the invention, as compared to unmodified cyclosporin or other cyclosporin derivatives, make these compounds promising therapeutic agents in the treatment or prevention of viral infections.

Thus, the present invention provides a cyclosporin derivative which is a compound of formula (I) or a pharmaceutically acceptable salt thereof, for use in the treatment or prevention of a viral infection: 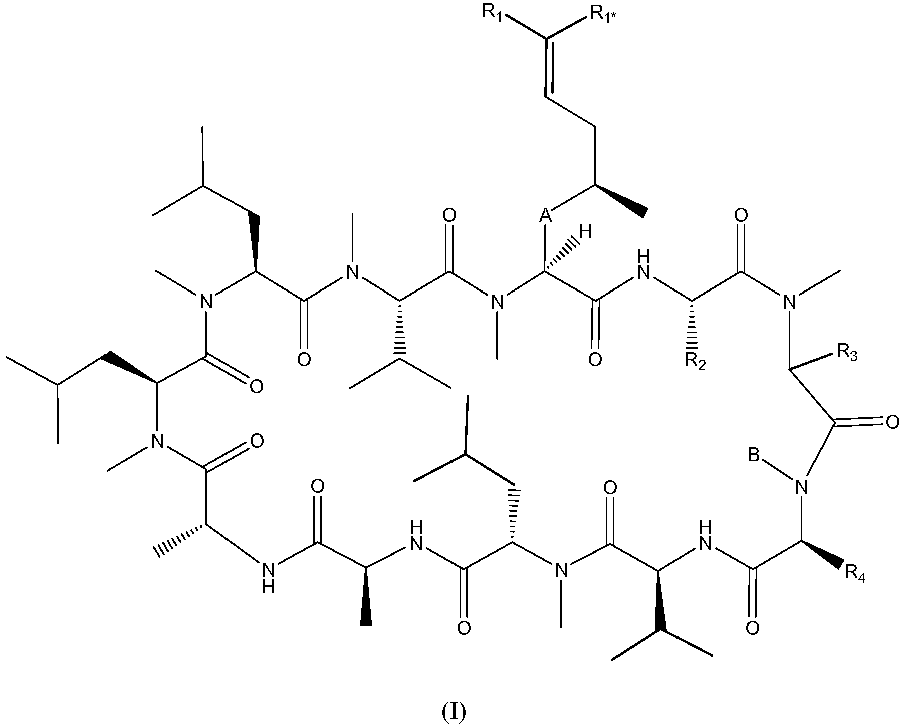 wherein:
A represents 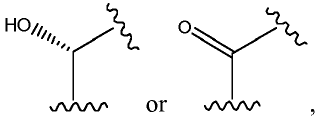 B represents methyl or ethyl,
R₂ represents ethyl or isopropyl,
R₄ represents -CH₂CH(CH₃)CH₃, -CH₂CH(CH₃)CH₂CH₃, -CH(CH₃)CH₃ or -CH(CH₃)CH₂CH₃, and
one of R₁ and R₁* represents hydrogen and the other represents methyl, and R₃ represents -L₃-G₃, wherein
L₃ represents a direct bond, a C₁-C₆ alkylene group or a C₂-C₆ alkenylene group, and
G₃ represents a hydrogen atom, a -COOR' group, or a phenyl moiety which is unsubstituted or substituted by one, two or three substituents selected from a halogen atom, a -COOR' group, a -CONR'R" group, a hydroxyl group, a C₁-C₆ alkyl group and a C₁-C₆ alkoxy group, wherein R' and R" are the same or different and represent hydrogen or a C₁-C₆ alkyl group, provided that G₃ does not represent a hydrogen atom when L₃ represents a direct bond or methylene.

The invention further provides a cyclosporin derivative which is a compound of formula (I*), or a pharmaceutically acceptable salt thereof: 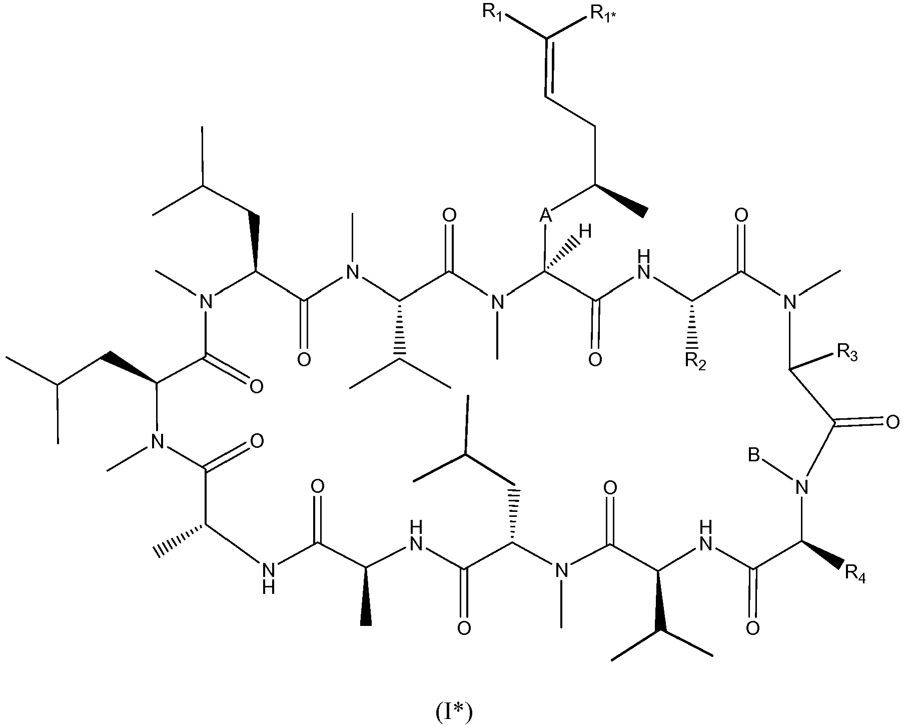 wherein:
A represents 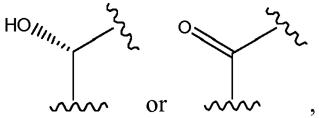 B represents methyl or ethyl,
R₂ represents ethyl or isopropyl,
R₄ represents -CH₂CH(CH₃)CH₃, -CH₂CH(CH₃)CH₂CH₃, -CH(CH₃)CH₃ or -CH(CH₃)CH₂CH₃, and
one of R₁ and R₁* represents hydrogen and the other represents methyl, and R₃ represents -L₃-G₃, wherein
L₃ represents a direct bond, a C₁-C₆ alkylene group or a C₂-C₆ alkenylene group, and
G₃ represents a hydrogen atom, a -COOR' group, or a phenyl moiety which is substituted by one, two or three substituents selected from a halogen atom, a -COOR' group, a -CONR'R" group, a hydroxyl group, a C₁-C₆ alkyl group and a C₁-C₆ alkoxy group, wherein R' and R" are the same or different and represent hydrogen or a C₁-C₆ alkyl group, provided that G₃ does not represent a hydrogen atom when L₃ represents a direct bond or methylene.

The invention further provides a pharmaceutical composition comprising a cyclosporin derivative which is a compound of formula (I*) or a or a pharmaceutically acceptable salt thereof and a pharmaceutically acceptable excipient, diluent or carrier.

The invention further provides a cyclosporin derivative which is a compound of formula (I*) or a pharmaceutically acceptable salt thereof, for use in the treatment of the human or animal body.

### DESCRIPTION OF THE FIGURES

Figure 1 shows the crystal structure of Compound 1 in complex with Cyclophilin A (CypA). The structure of cyclosporin is also superimposed. The extra moiety on position 3' in Compound 1 prevents binding to calcineurin due to steric clash.
Figure 2 shows the number of infected cells in samples of monocyte derived macrophages treated with Compound 1 and the number of infected cells in the control sample of monocyte derived macrophages. Compound 1 completely suppressed HIV-1 replication.
Figure 3 shows that the presence of Compound 1 elicited the production of IFN-β, as part of the innate immune response.
Figure 4 shows that after a single round infection in the presence of Compound 1, infection was 5-7 fold lower for monocyte derived macrophages.
Figure 5 shows the yield of CMV from cells treated in Example 4 with 5µM Compound 1 or DMSO vehicle, determined by plaque assay at various time points, after infection of HFF primary human fibroblasts.

### DETAILED DESCRIPTION OF THE INVENTION

In the compounds of the present invention, G₃ does not represent a hydrogen atom when L₃ represents a direct bond or methylene.

Compounds of formula (I*) in which G₃ does not represent an unsubstituted phenyl moiety have not been described previously. It is therefore preferred that G₃ represents a hydrogen atom, a -COOR' group, or a phenyl moiety which is substituted by one, two or three substituents selected from a halogen atom, a -COOR' group, a - CONR'R" group, a hydroxyl group, a C₁-C₆ alkyl group and a C₁-C₆ alkoxy group, wherein R' and R" are the same or different and represent hydrogen or a C₁-C₆ alkyl group.

Preferably, G₃ represents a hydrogen atom, a -COOR' group or phenyl moiety which is substituted by one or two, preferably one, substituents selected from a -COOR' group, a hydroxyl group and a C₁-C₆ alkoxy group, wherein R' represents hydrogen or a C₁-C₆ alkyl group.

More preferably, G₃ represents hydrogen atom, a -COOH group or a phenyl moiety which is substituted by one substituent which is a -COOR', more preferably a - COOH group. The substituent of the phenyl ring is preferably in the *meta* or *para* position, more preferably the *para* position. Thus, G₃ most preferably represents a hydrogen atom, a -COOH group or a phenyl group which is substituted by one -COOH moiety in the *para* position.

Typically, L₃ represents a C₁₋₃ alkylene moiety or a C₃-C₅ alkenylene group.

It is particularly preferred that L₃ represents a C₁₋₃ alkylene moiety and G₃ represents a phenyl moiety which is substituted by one substituent which is a -COOR' group, more preferably a -COOH group. The substituent of the phenyl ring is preferably in the *meta* or *para* position, more preferably the *para* position.

It is also particularly preferred that L₃ represents a C₃-C₅ alkenylene group and G₃ represents a hydrogen atom or a -COOR' group, more preferably a -COOH group.

It is further particularly preferred that L₃ represents a C₁₋₃ alkylene moiety and G₃ represents a -COOR' group, more preferably a -COOH group.

Typically, R' and R" are the same or different and represent hydrogen or methyl group.

Preferably, the compound of formula (I) has (R) stereochemistry at the 3' position, that is the position where the -L₃-G₃ moiety is attached.

Preferably, in the compound of formula (I), R₁ represents methyl and R₁* represents hydrogen.

In formula (I), L₃ typically represents a direct bond or a C₁-C₆ alkylene group; and G₃ represents a phenyl moiety which is unsubstituted or substituted by one, two or three substituents selected from a halogen atom, a -COOR' group, a -CONR'R" group, a hydroxyl group, a C₁-C₆ alkyl group and a C₁-C₆ alkoxy group, wherein R' and R" are the same or different and represent hydrogen or a C₁-C₆ alkyl group. Preferably L₃ represents a C₁₋₃ alkylene moiety, more preferably a methylene moiety. Preferably G₃ represents a phenyl moiety which is substituted by one or two, preferably one, substituents selected from a -COOR' group, a hydroxyl group and a C₁-C₆ alkoxy group, wherein R' represents hydrogen or a C₁-C₆ alkyl group. G₃ more preferably represents a phenyl moiety which is substituted by one substituent which is a -COOR', more preferably a -COOH group. The substituent is preferably in the *meta* or *para* position, more preferably the *para* position. Thus, in formula (I), G₃ most preferably represents a phenyl group which is substituted by one -COOH or one -COOMe moiety in the *para* position. Preferred examples are the compounds of formulae (I'A) and (I'B) or a pharmaceutically acceptable salt thereof:

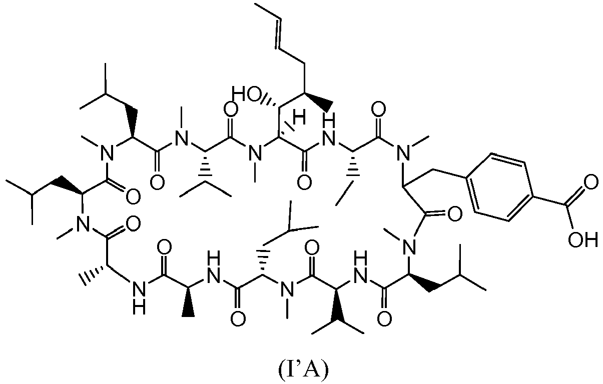

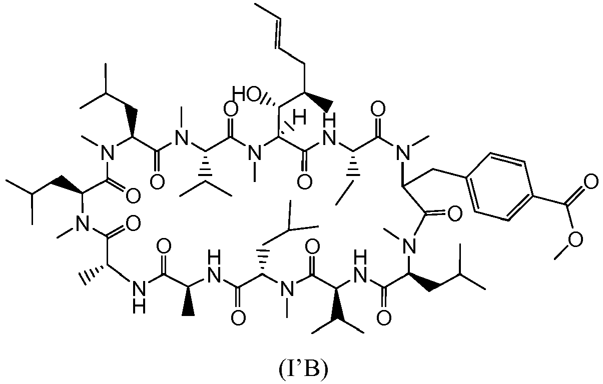

It is preferable that the compounds have (R) stereochemistry, and thus are formulae (I'C) or (I'D) or a pharmaceutically acceptable salt thereof:

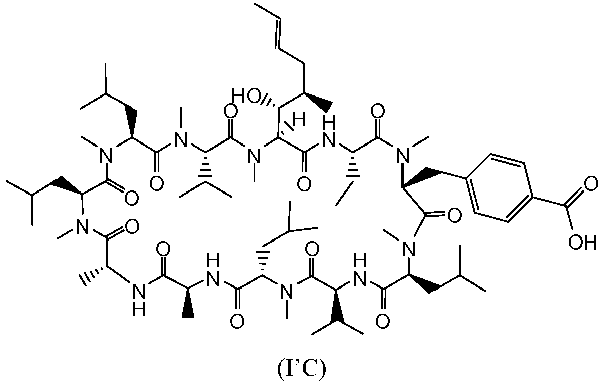

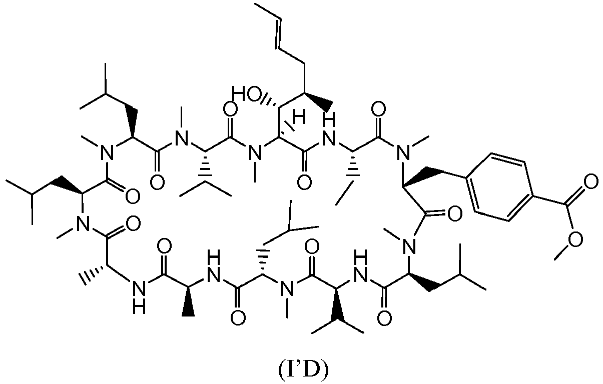

It is also particularly preferred in formula (I) that L₃ represents a C₃-C₅ alkenylene group and G₃ represents a hydrogen atom or a -COOR' group, more preferably a -COOH group. Preferred examples are the compound of formulae (I'E), (I'F) and (I'G) or pharmaceutically acceptable salts thereof:

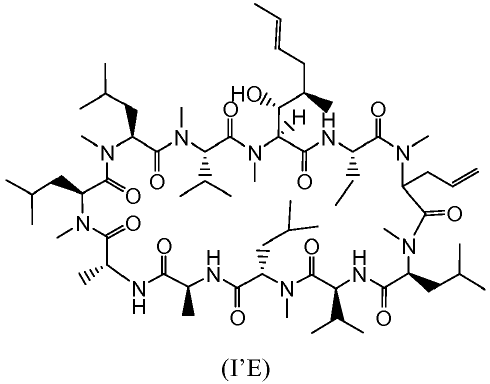

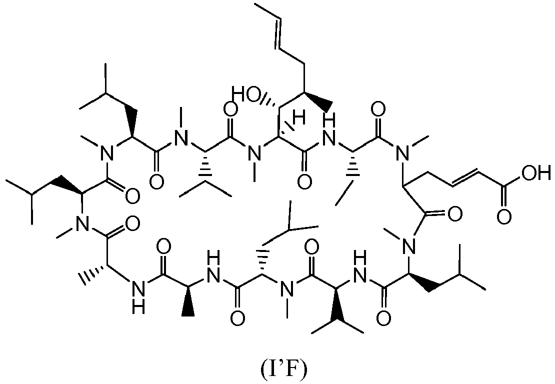

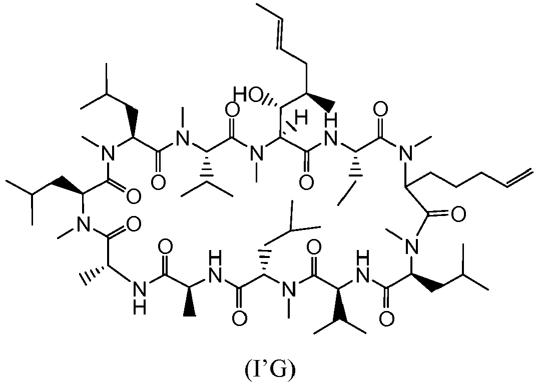

The residue at the 1 position of the cyclosporin derivatives of formula (I) and (I*) contains either a hydroxyl group or a ketone, depending on the identity of A. Thus, the residue at the 1 position is of formula (II) if A represents 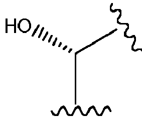 and of formula (II') if A represents

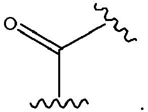

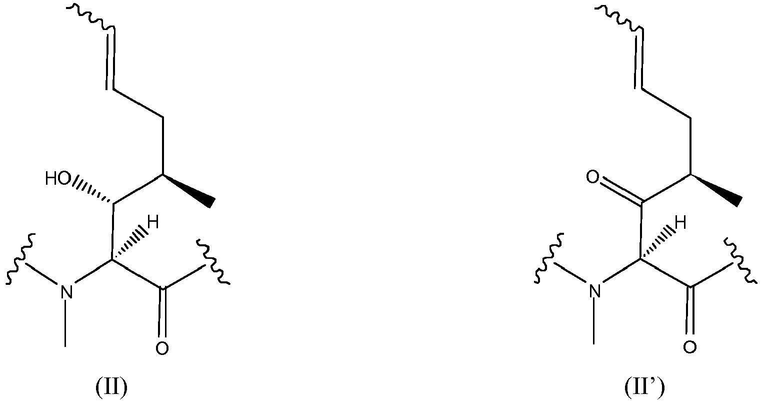

Typically, A represents

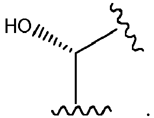

Typically, B represents methyl.

Typically, R₂ represents ethyl.

Typically, R₄ represents -CH₂CH(CH₃)CH₃.

Preferably, A represents 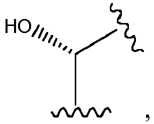 B represents methyl, R₂ represents ethyl and R₄ represents -CH₂CH(CH₃)CH₃.

As used herein, a C₁-C₆ alkyl group may be straight or branched and is typically a C₁-C₃ alkyl group. Preferred C₁-C₆ alkyl groups include, for example, methyl, ethyl, propyl, isopropyl, butyl, isobutyl, tert-butyl, pentyl and hexyl.

As used herein, a C₁-C₆ alkylene group is a said C₁-C₆ alkyl group which is divalent.

As used herein, a C₂-C₆ alkenylene group is a divalent moiety which may be straight or branched and is typically a C₃-C₅ alkenylene group. A C₂-C₆ alkenylene group typically contains one carbon-carbon double bond. The carbon-carbon double bond can have *cis* or *trans* configuration, with *trans* preferred.

As used herein, a C₁-C₆ alkoxy group is a said C₁-C₆ alkyl group which is attached to an oxygen atom. The alkoxy group is typically C₁-C₃ alkoxy group. Particularly preferred alkoxy groups include, for example, methyoxy, ethyoxy, propoxy, isopropoxy, butoxy, isobutoxy, tert-butoxy, pentoxy and hexoxy.

As used herein, a halogen is typically chlorine, fluorine, bromine or iodine and is preferably chlorine, bromine or fluorine.

As used herein, a pharmaceutically acceptable salt is typically a salt with a pharmaceutically acceptable base. Pharmaceutically acceptable bases include alkali metal (e.g. sodium or potassium) and alkali earth metal (e.g. calcium or magnesium) hydroxides and organic bases such as alkyl amines, aralkyl amines or heterocyclic amines.

The cyclosporin derivatives of the invention may be prepared by standard methods known in the art, typically starting from commercially available cyclosporin A.

The cyclosporin derivatives of the invention are useful in the treatment or prevention of a viral infection in a patient. The treatment or prevention of the viral infection is typically mediated by activation of innate pattern recognition receptors. Typically, the cyclosporin derivatives of the invention are useful in the treatment of a viral infection in a patient. Typically, the patient is a mammal, such as a human or a cat, preferably a human.

Typically, the viral infection is human immunodeficiency virus-1 (HIV-1), influenza virus, human cytomegalovirus (hCMV), hepatitis C virus (HCV), dengue virus, a vaccinia virus (such as Small Pox), feline immunodeficiency virus (FIV) or a corona virus (such as SARs). Preferably, the viral infection is human immunodeficiency virus-1 (HIV-1), influenza virus, human cytomegalovirus (hCMV) or hepatitis C virus (HCV), more preferably human immunodeficiency virus-1 (HIV-1).

It is particularly preferred that, the cyclosporin derivatives of the invention are for use in the treatment human immunodeficiency virus-1 (HIV-1), influenza virus, human cytomegalovirus (hCMV) or hepatitis C virus (HCV), more preferably human immunodeficiency virus-1 (HIV-1), in patients, typically human patients.

The cyclosporin derivatives of the invention may be administered to humans in various manners such as oral, rectal, vaginal, parenteral, intramuscular, intraperitoneal, intraarterial, intrathecal, intrabronchial, subcutaneous, intradermal, intravenous, nasal, buccal or sublingual routes of administration. The particular mode of administration and dosage regimen will be selected by the attending physician, taking into account a number of factors including the age, weight and condition of the patient.

The compound may be given to prevent viral (HIV) infection and administered via a medical device inserted into the vagina or rectum such that the device allows a slow release of the compound over a period of weeks or months such that the patient is protected from infection during the period that the device is in place.

The cyclosporin derivative is typically administered as a pharmaceutical composition, which generally comprises a derivative of the invention and a pharmaceutically acceptable excipient, diluent or carrier. Thus, pharmaceutical compositions that contain the cyclosporin derivatives of the invention will normally be formulated with an appropriate pharmaceutically acceptable excipient, carrier or diluent depending upon the particular mode of administration being used. For instance, parenteral formulations are usually injectable fluids that use pharmaceutically and physiologically acceptable fluids such as physiological saline, balanced salt solutions, or the like as a vehicle. Oral formulations, on the other hand, may be solids, e.g. tablets or capsules, or liquid solutions or suspensions.

Compositions may be formulated in unit dosage form, i.e., in the form of discrete portions containing a unit dose, or a multiple or sub-unit of a unit dose.

The amount of the cyclosporin derivative of the invention that is given to a patient will depend upon on the activity of the particular compound in question. Further factors include the condition being treated, the nature of the patient under treatment and the severity of the condition under treatment. The timing of administration of the compound should be determined by medical personnel. As a skilled physician will appreciate, and as with any drug, the compound may be toxic at very high doses. For example, the compound may be administered at a dose of from 0.01 to 30 mg/kg body weight, such as from 0.1 to 10 mg/kg, more preferably from 0.1 to 5 mg/kg body weight.

The cyclosporin derivatives of the invention may be given alone or in combination with one or more additional anti-viral agents, preferably one or more agents useful for treating human immunodeficiency virus-1 (HIV-1), influenza virus, human cytomegalovirus (hCMV), hepatitis C virus (HCV), dengue virus, vaccinia virus, feline immunodeficiency virus (FIV) or corona virus.

Anti-viral agents useful for treating HIV-1 include non-nucleoside reverse transcriptase inhibitors (NNRTIs), nucleoside analogue reverse transcriptase inhibitor (NRTIs) and nucleotide analog reverse-transcriptase inhibitors (NtRTIs). Preferred NRTIs include Zidovudine, Didanosine, Zalcitabine, Stavudine, Lamivudine, Abacavir, Emtricitabine, Entecavir and Apricitabine, Preferred NNRTIs include Efavirenz, Nevirapine, Delavirdine, Etravirine and Rilpivirine. Preferred NtRTIs include Tenofovir and Adefovir.

Anti-viral agents useful for treating influenza virus include (a) neuraminidase inhibitors such as oseltamivir and zanamivir, and (b) M2 protein inhibitors such as amantadine and rimantadine.

Anti-viral agents useful for treating human cytomegalovirus (hCMV) include human cytomegalovirus antibodies and antiviral agents such as Ganciclovir, Valganciclovir, Foscarnet and cidofovir.

Anti-viral agents useful for treating hepatitis C virus (HCV) include pegylated interferon alpha and ribavirin.

Anti-viral agents useful for treating vaccinia virus include cidofovir.

Anti-viral agents useful for treating feline immunodeficiency virus (FIV) include Lymphocyte T-Cell Immunomodulator.

The active ingredients are typically administered as a combined preparation.

Accordingly, the present invention also provides a combination comprising a cyclosporin derivative of the invention and one or more said additional anti-viral agents. The combination is typically for use in the treatment or prevention of said viral infection in a patient.

The invention further provides a cyclosporin derivative of the invention for use in the treatment or prevention of a viral infection in a patient, by co-administration with one or more said additional anti-viral agents. Co-administration can be simultaneous, concurrent, separate or sequential.

The invention further provides one or more additional said anti-viral agents, for use in the treatment or prevention of a said viral infection in a patient, by co-administration with a cyclosporin derivative of the invention. Co-administration can be simultaneous, concurrent, separate or sequential.

The present invention further provides a product comprising a cyclosporin derivative of the invention and one or more said additional anti-viral agents, as a combined preparation for simultaneous, concurrent, separate or sequential use in the treatment or prevention of a said viral infection in a patient.

The following Examples illustrate the invention.

### EXAMPLES

All commercially available solvents and reagents were used without further treatment as received unless otherwise noted. NMR spectra were measured with a Bruker DRX 500 or 600 MHz spectrometer; chemical shifts are expressed in ppm relative to TMS as an internal standard and coupling constants (J) in Hz. LC-MS spectra were obtained using a Waters ZQ2000 single quadrupole mass spectrometer with electrospray ionisation (ESI), using an analytical C4 column (Phenomenex Gemini, 50 x 3.6 mm, 5 µm) and an AB gradient of 50-95 % for B at a flow rate of 1 mL/minute, where eluent A was 0.1:5:95 formic acid/methanol/water and eluent B was 0.:5:95 formic acid/water/methanol. High resolution mass spectra were acquired on a Waters LCT time of flight mass spectrometer with electrospray ionisation (ESI) or chemical ionization (CI).

### Example 1 - Synthesis of Compound 1

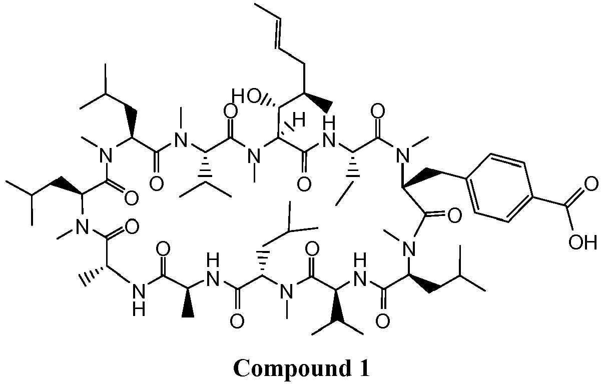

### Step 1 - alkylation

To LDA [1.8 M in hexane/ethylheptane] (39 mL,70 mmol) in THF (40 mL) at - 15°C was added LiCl solid and the solution stirred for 10 minutes (brown colour). Enantiomerically pure cyclosporin A (CsA) (6.0 g, 5 mmol) in THF (25 mL) was added drop-wise keeping the temperature at -15 to -20°C. The reaction was stirred 30 minutes at -20°C (brown colour). 4-bromomethylmethylbenzoate (2.0 g, 8.7 mmol) in THF (4 mL) was added dropwise keeping the temperature at -20°C to -15°C. The reaction was stirred at this temperature for 30 minutes (red colour). The temperature allowed to rise to -5°C then the reaction stopped by addition of 5% AcOH/ methanol (100 mL). The reaction was stirred overnight then a rotary evaporator used to remove THF and methanol. DCM (200 mL) and water (100 mL) were added. The DCM layer was separated. The water layer was extracted with DCM (2 x 50 mL). The combined DCM extracts were dried (Na₂SO₄). This gave 7.54g of crude CsA methyl ester. The product was purified using silica gel chromatography employing acetone/DCM to obtain a product of approximately 90% purity, 710 mg.

### Step 2 - Saponification

The CsA methyl ester (700 mg, 0.52 mmol) was dissolved in THF (7 mL) and methanol (2 mL) and lithium hydroxide solution (LiOH. H₂O, 210 mg, 5 mmol) dissolved in 5 mL water added. The reaction was stirred for 48 hours at room temperature and monitored by liquid chromatography-mass spectrometry (LCMS). A further equivalent of LiOH was added to drive the hydrolysis to completion. Compound 1 was isolated using ion exchange chromatography eluting with acetic acid/methanol/DCM. Yield 170 mg.

FAB+ve; Calc. *m*/*z* C₇₀H₁₁₇N₁₁O₁₄ (M+Na) 1358.86787, Found (M+Na) 1358.86447.

### Example 2 - crystal structure of Compound 1 in complex with Cyclophilin A (CypA)

CypA was expressed in Escherichia coli C41(DE3) cells (Lucigen) from tagless expression vector pOPT. Cells were grown overnight at 18 °C before being harvested, sonicated and purified by SP ion-exchange chromatography (GE Healthcare) followed by gel filtration. Crystals of Compound 1 in complex with CypA were grown at 17 °C in sitting drops. Protein solution (1mM each of CypA and Compound 1 in 20 mM Tris pH 8,50 mM NaCl, 1 mM DTT, 1 % DMSO) was mixed with reservoir solution (1 M LiCl, 0.1 M MES pH 6,30 % w/v PEG 6000) in a 1:1 mix, producing 0.15 mm x 0.10 mm x 0.10 mm crystals within 24 h.

Crystals were flash-frozen in liquid nitrogen before data collection using a Mar-345 detector. Crystal data and diffraction statistics are provided in Table 1 below.

**Table 1**

| **Data collection** | **Cyclophilin A/Compound 1 Complex** |
|---|---|
| Space Group | I222 |
| Cell Dimensions - a, b, c (Å) | 54.18, 64.60, 80.07 |
| Cell Dimensions - α, β, γ (°) | 90.00, 90.00, 90.00 |
| Resolution (Å) | 32.30-1.67 (1.76-1.67) * |
| R_{merge} | 0.061 (0.562) |
| I/σI | 17.1 (2.9) |
| Completeness (%) | 98.2 (91.1) |
| Redundancy | 6.0 (5.8) |

| **Refinement** | |
|---|---|
| Resolution (Å) | 1.67 |
| No. reflections | 15648 |
| R_{work}/R_{free} | 0.172/0.218 |
| No. atoms - Protein | 1250 |
| No. atoms - Ligand/ion | 97 |
| No. atoms - Water | 184 |
| B-factors - Protein | 19.485 |
| B-factors - Ligand/ion | 25.523 |
| B-factors - Water | 30.397 |
| R.m.s deviations - Bond lengths (Å) | 0.005 |
| R.m.s deviations - Bond angles (°) | 1.082 |

| | |
|---|---|
| *Highest resolution shell is shown in parenthesis. | |

Crystallographic analysis was performed using programs from the CCP4 suite13. Data were indexed and scaled in MOSFLM and SCALA, respectively. The structure of CsA:CypA (pdb 1CWA14) was used as a search model. Structures were refined in REFMAC and Coot13. Structureswere created using PyMol.

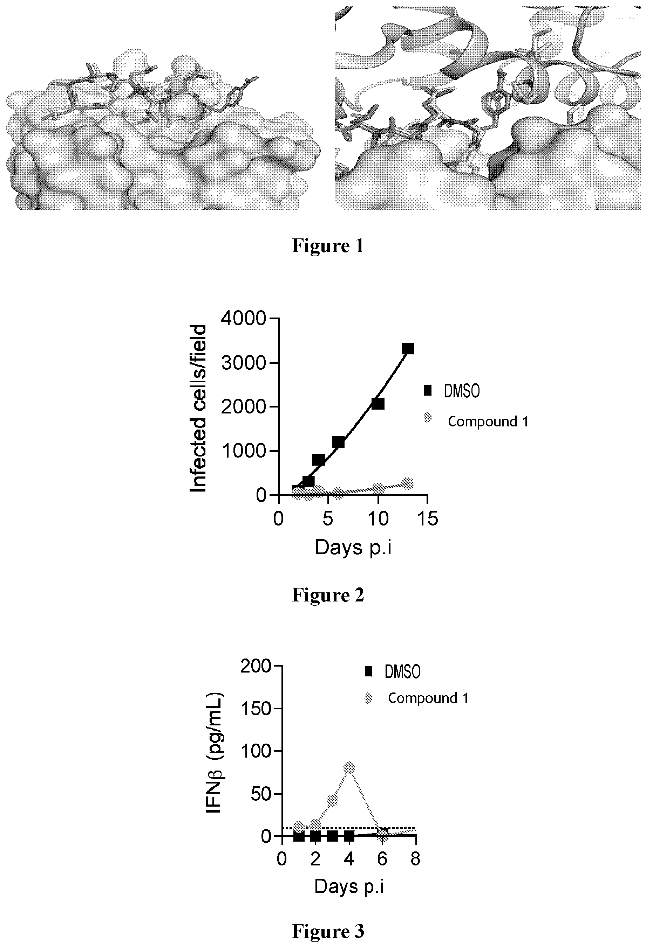

The crystal structure of Compound 1 in complex with CypA is shown in Figure 1. The structure of CsA is superposed. The extra moiety on position 3' in Compound 1 prevents binding to calcineurin due to steric clash. The means that Compound 1 is non-immunosuppressive.

### Example 3 - treatment of infected HIV-1 monocytes with Compound 1

### Monocyte preparation

Primary monocyte-derived macrophages (MDM) were prepared from fresh blood from healthy volunteers. Peripheral blood mononuclear cells were isolated by Ficoll-Hypaque (Axis-Shield) density centrifugation. The isolated cells were washed with PBS and plated in RPMI (Invitrogen) supplemented with 10% heat-inactivated autologous human serum (HS) and 40ng/ml macrophage colony stimulating factor (M-CSF) (R&D systems). The medium was then refreshed after 3 days (RPMI 1640 with 10% HS), removing any remaining non-adherent cells. After 6 days, media was replenished with RPMI containing 5% type AB HS (Sigma-Aldrich-Aldrich). Replicate experiments were performed with cells derived from different donors.

### Virus production

Virus particles were produced by transient transfection of HEK293T cells. 35µg of molecular clone DNA; NL4.3Bal was transfected using Fugene 6 transfection reagent (Promega). Virus supernatants were harvested 48hr, 72hr and 96hr post transfection. All virus suspensions were filtered and ultracentrifuged through a 20% sucrose buffer and resuspended in RPMI 1640 with 5% HS, for subsequent infection of MDM. All virus preparations were quantified by reverse transcriptase (RT) enzyme linked immunosorbant assay (ELISA) (Roche).

### Infection and stimulation

MDM were infected with 100 pg RT/well (MOI 0.2) in 48-well plates and subsequently fixed and stained using CA specific antibodies (EVA365 & 366 National Institute of Biological Standards AIDS Reagents Programme) and a secondary antibody linked to beta galactosidase. Infection was performed in DMSO in the presence or absence of 10µM Compound 1. Cells were stained for Gag p24 at specific time points after infection and infected colonies counted. During the time course, supernatants were collected for IFN-β ELISA (PBL Interferon Source) according to manufacturer's instructions.

### Results

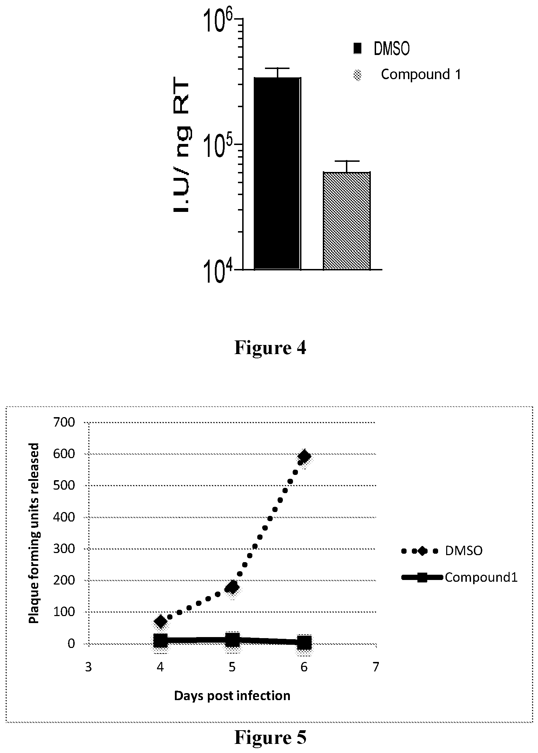

Figure 2 shows the number of infected cells in samples of MDM treated with Compound 1 and the number of infected cells in the control sample. Compound 1 completely suppressed HIV-1 replication and elicited the production of IFN-β, as shown in Figure 3. Further, after single round infection in the presence of Compound 1, infection was 5-7 fold lower on MDM at MOI of 0.1-1 as show in Figure 4.

### Example 4 - inhibition of human cytomegalovirus

Human cytomegalovirus (hCMV, strain Towne RC256) was used to infect primary human foreskin fibroblasts (HFF) at a multiplicity of 0.01 particle forming units per cell in the presence of either 5µM Compound 1 in DMSO or DMSO vehicle as a control. The infected cells were then incubated for time points as shown in Figure 5 before collection of the supernatants.

Progeny virus was detected by plaque assay of supernatants on fresh HFF. 7 days later, HCMV plaques were revealed by immunocytochemical staining using the cytomegalovirus early antigen specific primary mouse monoclonal antibody HCMV3, and goat anti mouse antibody conjugated to alkaline phosphatase, with the colorimetric substrate fast red. Plaque forming units of HCMV are plotted against time in Figure 5

### Example 5 - inhibition of feline immunodeficiency virus

Feline immunodeficiency virus (FIV) is used to infect a feline T cell line or a feline fibroblast line at a multiplicity of 0.01 particle forming units per cell in the presence of either 5µM of the test compound of the invention in DMSO or DMSO vehicle as a control. The infected cells are then incubated for various time points before fixation with paraformaldehyde and enumeration of the infected cells.

To detect infected cells the cells are stained with anti-FIV antibody and secondary antibody labeled with a fluorescent marker. Fluorescent cells are then measured by flow cytometry. The number of infected cells are plotted against time.

### Example 6 - inhibition of human influenza A virus

Human influenza A virus (IAV) was used to infect human A549 cells at a multiplicity of 0.01 infectious unit per cell in the presence of either 5µM of the test compound of the invention in DMSO or DMSO vehicle as a control. The infected cells are then incubated for various time points before collection of the supernatants. Progeny virus are detected by plaque assay of supernatants on fresh A549 cells. IAV plaques are revealed by immunocytochemical staining using IAV specific primary antibody, and secondary antibody conjugated to alkaline phosphatase, with the colorimetric substrate fast red. Plaque forming units of IAV are plotted against time.

### Example 7 - inhibition of vaccinia virus

To assess the impact of compounds of the invention on vaccinia virus (VACV) infection, primary human foreskin fibroblasts (HFFs) are infected at an MOI of 1 with recombinant VACV expressing EGFP under the control of an early/late viral promoter in the presence of increasing concentrations of the compound of the invention. At six hours post infection cells are harvested and scored for EGFP by flow cytometry. In order to determine if the compound impacted VACV production, HFFs are infected with wildtype virus at an MOI of 1. Twenty-four hours post infection cells are harvested and virus released by three cycles of freeze/thawing. Virus production is quantified by plaque assay on BSC40 green monkey kidney cells. For all experiments samples treated with vehicle alone (DMSO) are used as controls.

### Example 8 - inhibition of HCV

Inhibition of HCV by the compounds of the invention is tested using subgenomic replicons derived from HCV. The subgenomic replicons encode all proteins required for RNA replication and a marker gene such as luciferase or green fluorescent protein (GFP). Cells expressing the subgenomic replicon are treated with the test compounds for various time points between 4 and 48 hours. At these time points replication are assessed by measurement of the marker (luciferase or GFP).

### Example 9 - inhibition of Dengue virus

Inhibition of Dengue virus by the compounds of the invention is tested using subgenomic replicons derived from Dengue virus. The subgenomic replicons encode all proteins required for RNA replication and a marker gene such as luciferase or green fluorescent protein (GFP). Cells expressing the subgenomic replicon are treated with the test compounds for various time points between 4 and 48 hours. At these time points replication are assessed by measurement of the marker (luciferase or GFP).

### Example 10 - Synthesis of Compound 2 ([Sar-Allyl]³ CsA)

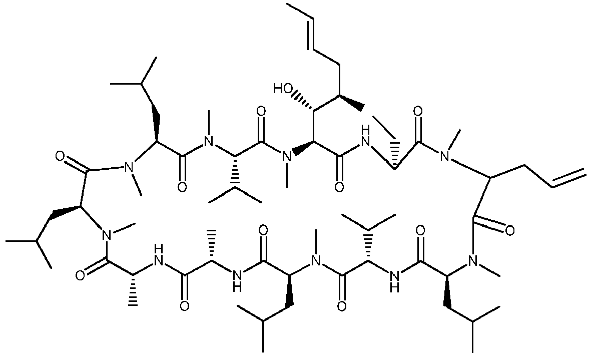

To a stirred solution of 1.8M lithium diisopropylamide (39 mL, 70 mmol) in anhydrous THF at -10 °C was added dropwise a cooled solution of Cyclosporin A (6 g, 5 mmol) and lithium chloride (3.81 g, 89 mmol) in THF. The mixture was stirred at this temperature for an hour before the dropwise addition of a solution of allyl bromide (0.755 ml, 8.7 mmol) in THF. After a further 3 hours stirring at -5 °C the reaction was quenched by the addition of 5% acetic acid in methanol solution.

The mixture was concentrated under reduced pressure before dissolution in DCM and water. The DCM layer was separated and the aqueous layer was extracted twice with DCM. The organic fractions were combined, dried over magnesium sulphate and concentrated under reduced pressure. The product was purified by flash silica chromatography (0-20% acetone:DCM gradient) to give Compound 2 as an off-white solid (1.144g, 18%).

HRMS (*m*/*z*): [MH]⁺ calcd. for C₆₅H₁₁₅N₁₁O₁₂, 1242.88; found 1242.89.

### Example 11 - Synthesis of Compound 3 ([Sar-(E)-But-2-enoic acid]³ CsA)

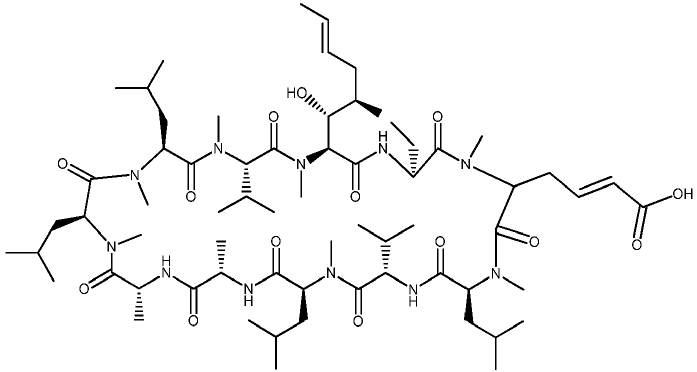

To a solution of Compoun2 (3-allyl Cyclosporin A) (75 mg, 0.06 mmol) in DCM (2 mL) was added acrylic acid (5 µl, 0.072 mmol) and Hoveyda-Grubbs 2n^{d} generation catalyst (7 mg, 0.01 mmol, 17mol%). The reaction was stirred in the microwave at 90 °C for 30 minutes and then allowed to cool. Triethylamine was added to the mixture and then stirred overnight with excess P(CH₂OH)₃ to coordinate the ruthenium catalyst. This was then washed away with brine and water before the mixture was then passed through a Stratospheres SPE, PL Thiol MP SPE cartridge (polymer Lab, Varian Inc) to remove any remaining catalyst. The crude product was purified by flash silica chromatography (40:8:1 DCM: MeOH: NH3) to give Compound 3 as an off-white solid (45 mg, 53%).

HRMS (*m*/*z*): [MH]⁺ calcd. for C₆₆H₁₁₅N₁₁O₁₄, 1286.68 found 1286.94.

### Example 12 - Synthesis of Compound 4 ([Sar-methyl-4-methylbenzoate]³ CsA)

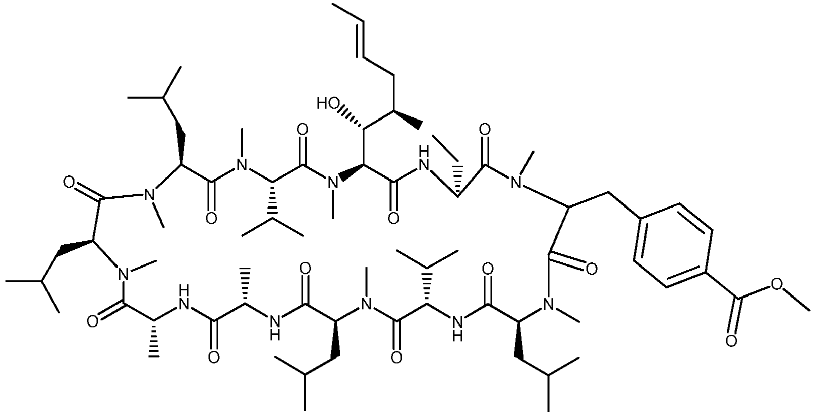

To a solution of the benzoic acid derivative (25 mg, 0.019 mmol) in methanol (2 mL) was added a catalytic amount of concentrated sulphuric acid. The reaction was heated to reflux for an hour and then allowed to cool before neutralising with 10% NaHCO₃ solution. The product was extracted with DCM, washed with water and concentrated under reduced pressure. The crude product was purified by preparative HPLC chromatography (MeOH:H₂0:formic acid) to give Compound 4 as a white solid (5 mg, 20%).

LC-MS [MH]⁺ calcd. for C₇₁H₁₁₉N₁₁O₁₄, 1350.77; found 1350.50

### Example 13 - Synthesis of Compound 5 ([Sar-Pent-4-enyl]³ CsA)

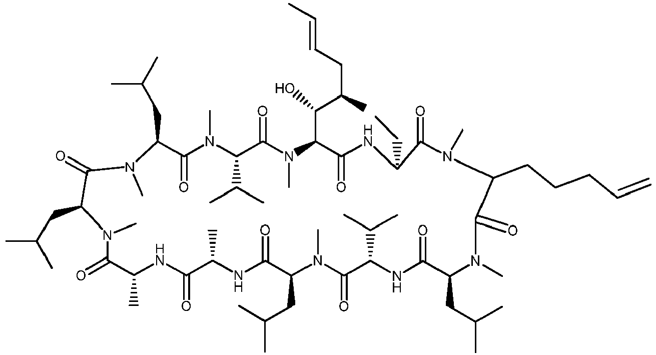

To a stirred solution of 2M lithium diisopropylamide in heptane/THF (3.75 mL, 7.5 mmol) in anhydrous THF at -10 °C was added dropwise a cooled solution of Cyclosporin A (0.6 g, 0.5 mmol) in THF. The mixture was stirred at this temperature for an hour before the dropwise addition of a solution of 5-bromopent-1-ene (0.190 ml, 1.25 mmol) in THF. After a further 3 hours stirring at -5 °C the reaction was quenched by the addition of 5% acetic acid in methanol solution.

The mixture was concentrated under reduced pressure before dissolution in DCM and water. The DCM layer was separated and the aqueous layer was extracted twice with DCM. The organic fractions were combined, dried over magnesium sulphate and concentrated under reduced pressure. The product was purified by flash silica chromatography (0-20% acetone:DCM gradient) to give Compound 5 as an off-white solid (0.065g, 10%).

LC-MS [MH]⁺ calcd. for C₆₇H₁₁₉N₁₁O₁₂, 1270.73; found 1270.60.

## Claims

1. A cyclosporin derivative which is a compound of formula (I), or a pharmaceutically acceptable salt thereof, for use in the treatment or prevention of a viral infection: 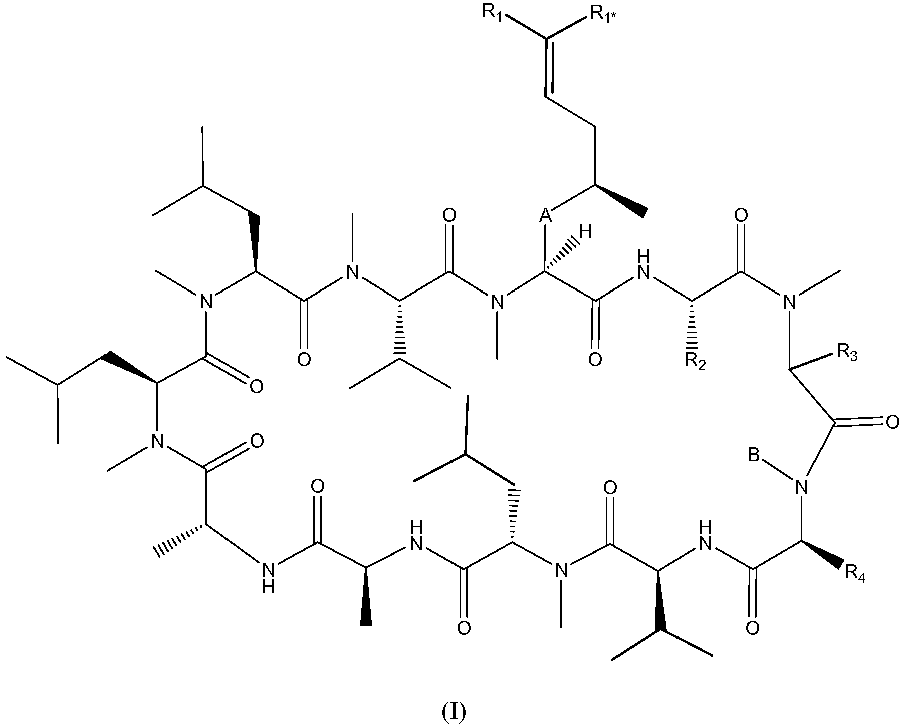 wherein:
A represents 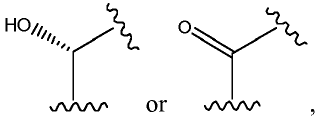
B represents methyl or ethyl,
R₂ represents ethyl or isopropyl,
R₄ represents -CH₂CH(CH₃)CH₃, -CH₂CH(CH₃)CH₂CH₃, -CH(CH₃)CH₃ or -CH(CH₃)CH₂CH₃, and
one of R₁ and R₁* represents hydrogen and the other represents methyl, and R₃ represents -L₃-G₃, wherein
L₃ represents a direct bond, a C₁-C₆ alkylene group or a C₂-C₆ alkenylene group; and
G₃ represents a hydrogen atom, a -COOR' group, or a phenyl moiety which is unsubstituted or substituted by one, two or three substituents selected from a halogen atom, a -COOR' group, a -CONR'R" group, a hydroxyl group, a C₁-C₆ alkyl group and a C₁-C₆ alkoxy group, wherein R' and R" are the same or different and represent hydrogen or a C₁-C₆ alkyl group, provided that G₃ does not represent a hydrogen atom when L₃ represents a direct bond or methylene.

2. A cyclosporin derivative for use according to claim 1, wherein the viral infection is human immunodeficiency virus-1 (HIV-1), influenza virus, human cytomegalovirus (hCMV), hepatitis C virus (HCV), dengue virus, vaccinia virus, feline immunodeficiency virus (FIV) or corona virus.

3. A cyclosporin derivative for use according to claim 2, wherein the viral infection is human immunodeficiency virus-1 (HIV-1), influenza virus, human cytomegalovirus (hCMV) or hepatitis C virus (HCV).

4. A cyclosporin derivative for use according to claim 3, wherein the viral infection is human immunodeficiency virus-1 (HIV-1) or human cytomegalovirus (hCMV).

5. A cyclosporin derivative for use according to claim 4, wherein the viral infection is human immunodeficiency virus-1 (HIV-1).

6. A cyclosporin derivative for use according to any one of the preceding claims, which is for use in treatment of the viral infection.

7. A cyclosporin derivative for use according to any one of the preceding claims, wherein:
A represents 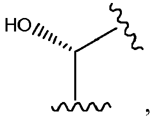
B represents methyl,
R₂ represents ethyl, and
R₄ represents -CH₂CH(CH₃)CH₃.

8. A cyclosporin derivative for use according to any one of the preceding claims, wherein L₃ represents a C₁₋₃ alkylene moiety or a C₃-C₅ alkenylene group.

9. A cyclosporin derivative for use according to any one of the preceding claims, wherein G₃ represents a hydrogen atom, a -COOR' group, or a phenyl moiety which is substituted by one, two or three substituents selected from a halogen atom, a -COOR' group, a -CONR'R" group, a hydroxyl group, a C₁-C₆ alkyl group and a C₁-C₆ alkoxy group, wherein R' and R" are the same or different and represent hydrogen or a C₁-C₆ alkyl group.

10. A cyclosporin derivative which is a compound of formula (I*), or a pharmaceutically acceptable salt thereof: 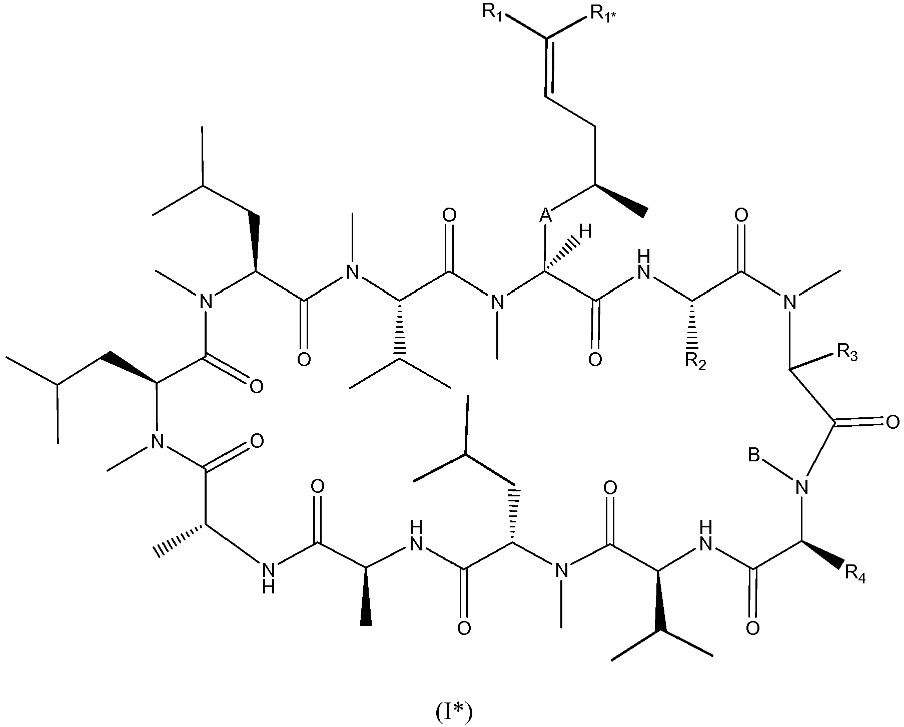 wherein:
A, B, R₂ and R₄ are as defined in claim 1 or 7,
R₁, R₁* and R₃ are as defined in claim 1
L₃ is as defined in claim 1 or 8, and
G₃ represents a hydrogen atom, a -COOR' group, or a phenyl moiety which is substituted by one, two or three substituents selected from a halogen atom, a -COOR' group, a -CONR'R" group, a hydroxyl group, a C₁-C₆ alkyl group and a C₁-C₆ alkoxy group, wherein R' and R" are the same or different and represent hydrogen or a C₁-C₆ alkyl group, provided that G₃ does not represent a hydrogen atom when L₃ represents a direct bond or methylene.

11. A pharmaceutical composition comprising a cyclosporin derivative as defined in claim 10 and a pharmaceutically acceptable excipient, diluent or carrier.

12. A cyclosporin derivative as defined in claim 10, for use in the treatment of the human or animal body.

## Patentansprüche

1. Cyclosporinderivat, das eine Verbindung der Formel (I) ist, oder ein pharmazeutisch annehmbares Salz davon zur Verwendung bei der Behandlung oder Prävention einer Virusinfektion: 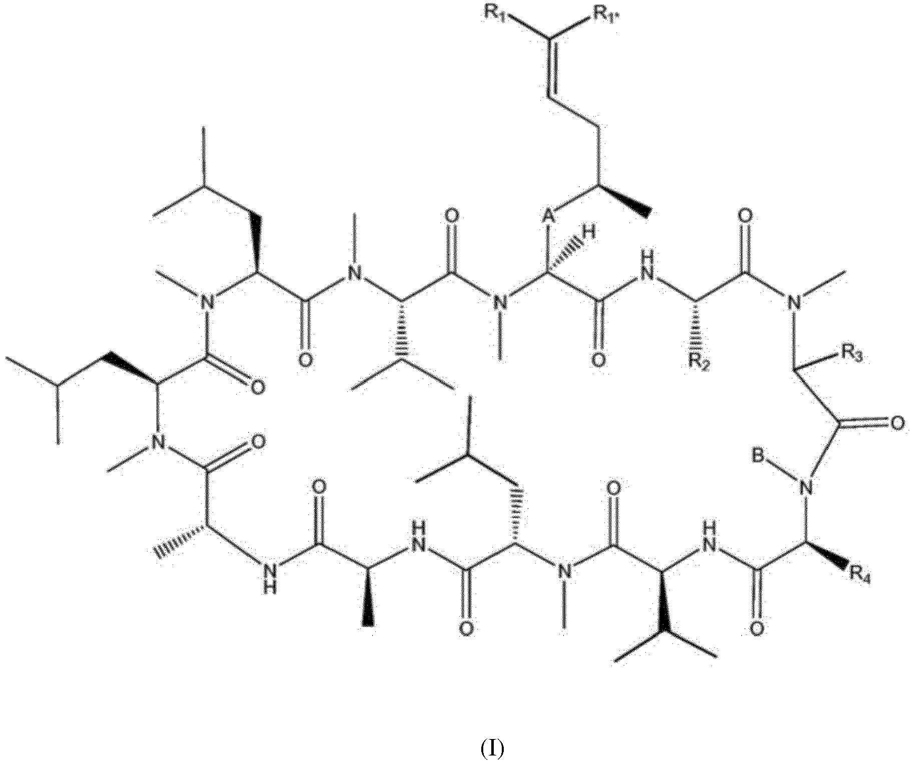 wobei:
A 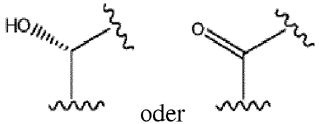 darstellt,
B Methyl oder Ethyl darstellt,
R₂ Ethyl oder Isopropyl darstellt,
R₄ -CH₂CH(CH₃)CH₃, -CH₂CH(CH₃)CH₂CH₃, -CH(CH₃)CH₃ oder - CH(CH₃)CH₂CH₃ darstellt, und
eines von R₁ und R₁* Wasserstoff darstellt und das andere Methyl darstellt, und R₃-L₃-G₃ darstellt, wobei
L₃ eine direkte Bindung, eine C₁-C₆-Alkylengruppe oder eine C₂-C₆-Alkenylengruppe darstellt; und
G₃ ein Wasserstoffatom, eine -COOR'-Gruppe oder eine Phenyleinheit darstellt, unsubstituiert oder substituiert durch ein, zwei oder drei Substituenten, die aus einem Halogenatom, einer -COOR'-Gruppe, einer -CONR'R"-Gruppe, einer Hydroxylgruppe, einer C₁-C₆-Alkylgruppe und einer C₁-C₆-Alkoxygruppe ausgewählt sind, wobei R' und R" gleich oder unterschiedlich sind und Wasserstoff oder eine C₁-C₆-Alkylgruppe darstellen, mit der Maßgabe, dass G₃ kein Wasserstoffatom darstellt, wenn L₃ eine direkte Bindung oder Methylen darstellt.

2. Cyclosporinderivat zur Verwendung nach Anspruch 1, wobei die Virusinfektion humanes Immundefizienzvirus 1 (HIV-1), Influenzavirus, humanes Cytomegalievirus (hCMV), Hepatitis-C-Virus (HCV), Denguevirus, Vacciniavirus, felines Immundefizienzvirus (FIV) oder Coronavirus ist.

3. Cyclosporinderivat zur Verwendung nach Anspruch 2, wobei die Virusinfektion humanes Immundefizienzvirus 1 (HIV-1), Influenzavirus, humanes Cytomegalievirus (hCMV) oder Hepatitis-C-Virus (HCV) ist.

4. Cyclosporinderivat zur Verwendung nach Anspruch 3, wobei die Virusinfektion humanes Immundefizienzvirus 1 (HIV-1) oder humanes Cytomegalievirus (hCMV) ist.

5. Cyclosporinderivat zur Verwendung nach Anspruch 4, wobei die Virusinfektion humanes Immundefizienzvirus 1 (HIV-1) ist.

6. Cyclosporinderivat zur Verwendung nach einem der vorstehenden Ansprüche zur Verwendung bei der Behandlung der Virusinfektion.

7. Cyclosporinderivat zur Verwendung nach einem der vorstehenden Ansprüche, wobei:
A 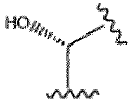 darstellt,
B Methyl darstellt,
R₂ Ethyl darstellt, und
R₄ -CH₂CH(CH₃)CH₃ darstellt.

8. Cyclosporinderivat zur Verwendung nach einem der vorstehenden Ansprüche, wobei L₃ eine C₁-₃-Alkyleneinheit oder eine C₃-C₅-Alkenylengruppe darstellt.

9. Cyclosporinderivat zur Verwendung nach einem der vorstehenden Ansprüche, wobei G₃ ein Wasserstoffatom, eine -COOR'-Gruppe oder eine Phenyleinheit darstellt,
substituiert durch einen, zwei oder drei Substituenten, die aus einem Halogenatom, einer -COOR'-Gruppe, einer -CONR'R"-Gruppe, einer Hydroxylgruppe, einer C₁-C₆-Alkylgruppe und einer C₁-C₆-Alkoxygruppe ausgewählt sind, wobei R' und R" gleich oder unterschiedlich sind und Wasserstoff oder eine C₁-C₆-Alkylgruppe darstellen.

10. Cyclosporinderivat, das eine Verbindung der Formel (I*) ist, oder ein pharmazeutisch annehmbares Salz davon: 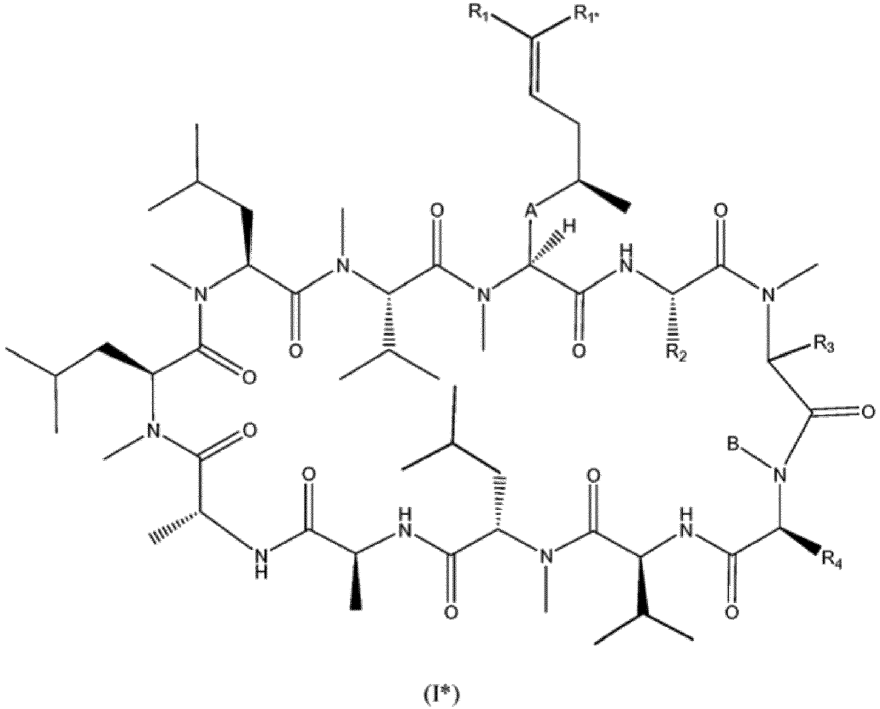 wobei:
A, B, R₂ und R₄ wie in Anspruch 1 oder 7 definiert sind,
R₁, R₁* und R₃ wie in Anspruch 1 definiert sind,
L₃ wie in Anspruch 1 oder 8 definiert ist, und
G₃ ein Wasserstoffatom, eine -COOR'-Gruppe oder eine Phenyleinheit darstellt, substituiert durch ein, zwei oder drei Substituenten substituiert, die aus einem Halogenatom, einer -COOR'-Gruppe, einer -CONR'R"-Gruppe, einer Hydroxylgruppe, einer C₁-C₆-Alkylgruppe und einer C₁-C₆-Alkoxygruppe ausgewählt sind, wobei R' und R" gleich oder unterschiedlich sind und Wasserstoff oder eine C₁-C₆-Alkylgruppe darstellen, mit der Maßgabe, dass G₃ kein Wasserstoffatom darstellt, wenn L₃ eine direkte Bindung oder Methylen darstellt.

11. Pharmazeutische Zusammensetzung, die ein Cyclosporinderivat nach Anspruch 10 und einen pharmazeutisch annehmbaren Exzipienten, ein pharmazeutisch annehmbares Verdünnungsmittel oder einen pharmazeutisch annehmbaren Trägerstoff umfasst.

12. Cyclosporinderivat nach Anspruch 10 zur Verwendung bei der Behandlung des menschlichen oder tierischen Körpers.

## Revendications

1. Dérivé de cyclosporine, étant un composé de formule (I), ou un sel pharmaceutiquement acceptable de celui-ci, destiné à être utilisé dans le traitement ou la prévention d'une infection virale : 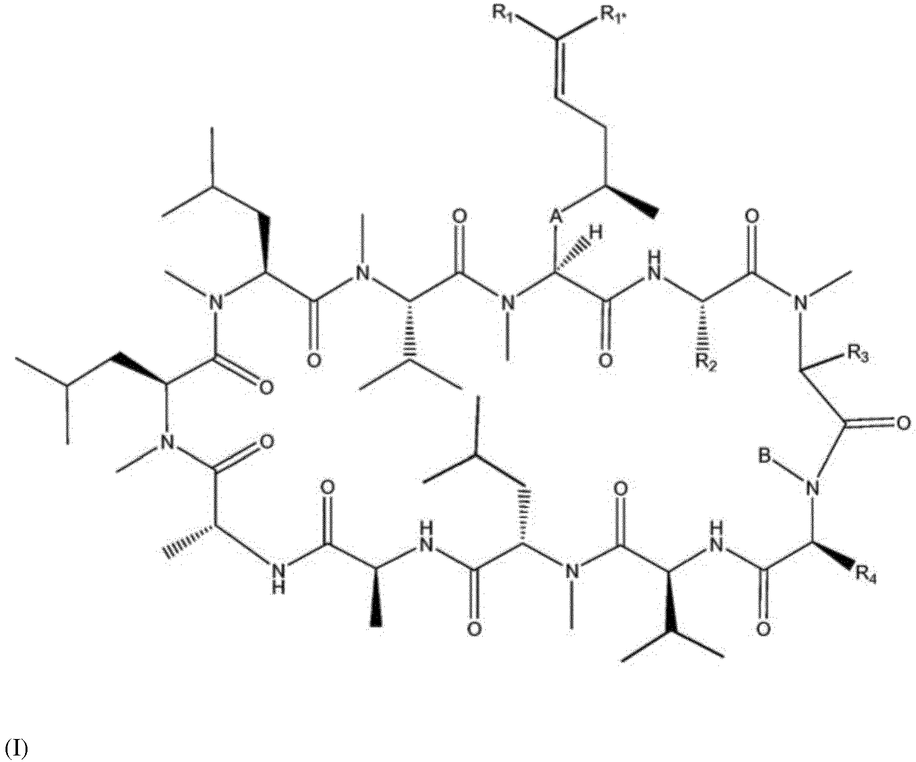 dans lequel :
A représente 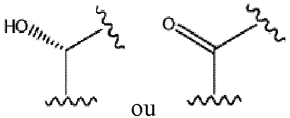
B représente un radical méthyle ou éthyle,
R₂ représente un radical éthyle ou isopropyle,
R₄ représente un radical -CH₂CH(CH₃)CH₃, -CH₂CH(CH₃)CH₂CH₃, -CH(CH₃)CH₃ ou -CH(CH₃)CH₂CH₃, et
l'un parmi R₁ et R₁* représente un radical hydrogène et l'autre un radical méthyle, et R₃ représente un radical -L₃-G₃, dans lequel
L₃ représente une liaison directe, un groupe alkylène C₁-C₆ ou un groupe alcénylène C₂-C₆ ; et
G₃ représente un atome d'hydrogène, un groupe -COOR', ou une fraction phényle, non substitué(e) ou substitué(e) par un, deux ou trois substituants sélectionnés à partir d'un atome d'halogène, d'un groupe -COOR', d'un groupe -CONR'R", d'un groupe hydroxyle, d'un groupe alkyle C₁-C₆ et d'un groupe alcoxyle C₁-C₆, dans lequel R' et R" sont identiques ou différents et représentent un hydrogène ou un groupe alkyle C₁-C₆, à condition que G₃ ne représente pas un atome d'hydrogène lorsque L₃ représente une liaison directe ou du méthylène.

2. Dérivé de cyclosporine destiné à être utilisé selon la revendication 1, dans lequel l'infection virale est le virus de l'immunodéficience humaine de type 1 (VIH-1), le virus de la grippe, le cytomégalovirus humain (hCMV), le virus de l'hépatite C (HCV), le virus de la dengue, le virus de la vaccine, le virus de l'immunodéficience féline (FIV) ou le coronavirus.

3. Dérivé de cyclosporine destiné à être utilisé selon la revendication 2, dans lequel l'infection virale est le virus de l'immunodéficience humaine de type 1 (VIH-1), le virus de la grippe, le cytomégalovirus humain (hCMV) ou le virus de l'hépatite C (HCV).

4. Dérivé de cyclosporine destiné à être utilisé selon la revendication 3, dans lequel l'infection virale est le virus de l'immunodéficience humaine de type 1 (VIH-1) ou le cytomégalovirus humain (hCMV).

5. Dérivé de cyclosporine destiné à être utilisé selon la revendication 4, dans lequel l'infection virale est le virus de l'immunodéficience humaine de type 1 (VIH-1).

6. Dérivé de cyclosporine destiné à être utilisé selon l'une quelconque des revendications précédentes, destiné à être utilisé dans le traitement d'une infection virale.

7. Dérivé de cyclosporine destiné à être utilisé selon l'une quelconque des revendications précédentes, dans lequel :
A représente 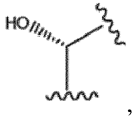
B représente un radical méthyle,
R₂ représente un radical éthyle, et
R₄ représente un radical -CH₂CH(CH₃)CH₃.

8. Dérivé de cyclosporine destiné à être utilisé selon l'une quelconque des revendications précédentes, dans lequel L₃ représente une fraction alkylène C₁₋₃ ou un groupe alcénylène C₃-C₅.

9. Dérivé de cyclosporine destiné à être utilisé selon l'une quelconque des revendications précédentes, dans lequel G₃ représente un atome d'hydrogène, un groupe - COOR', ou une fraction phényle,
substitué(e) par un, deux ou trois substituants sélectionnés à partir d'un atome d'halogène, d'un groupe -COOR', d'un groupe -CONR'R", d'un groupe hydroxyle, d'un groupe alkyle C₁-C₆ et d'un groupe alcoxyle C₁-C₆, dans lequel R' et R" sont identiques ou différents et représentent un hydrogène ou un groupe alkyle C₁-C₆.

10. Dérivé de cyclosporine, étant un composé de formule (I), ou un sel pharmaceutiquement acceptable de celui-ci : 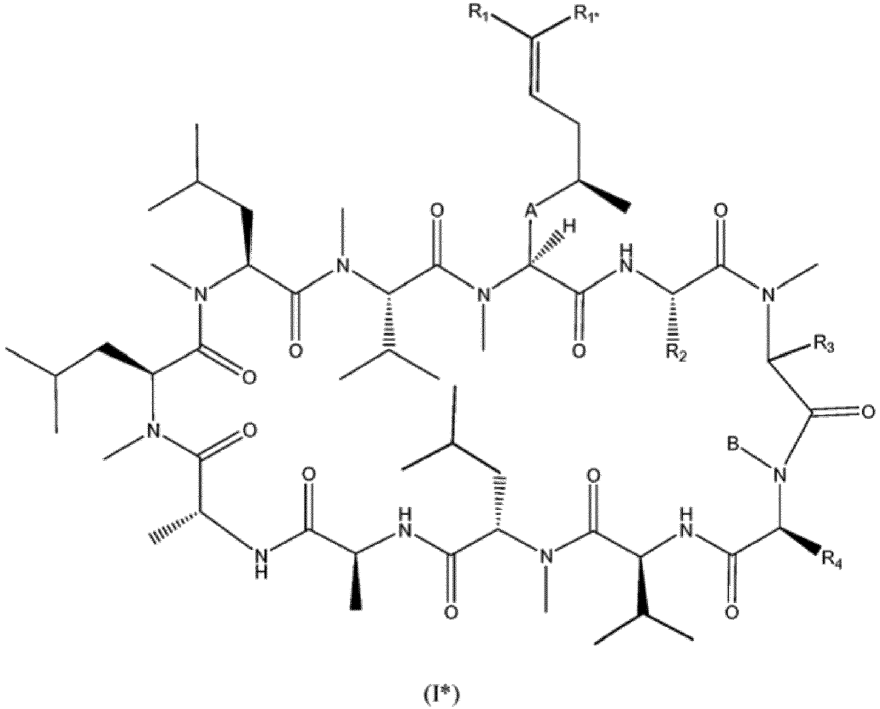 dans lequel :
A, B, R₂ et R₄ sont tels que définis dans la revendication 1 ou 7,
R₁, R₁* et R₃ sont tels que définis dans la revendication 1
L₃ est tel que défini dans la revendication 1 ou 8, et
G₃ représente un atome d'hydrogène, un groupe -COOR', ou une fraction phényle, substitué(e) par un, deux ou trois substituants sélectionnés à partir d'un atome d'halogène, d'un groupe -COOR', d'un groupe -CONR'R", d'un groupe hydroxyle, d'un groupe alkyle C₁-C₆ et d'un groupe alcoxyle C₁-C₆, dans lequel R' et R" sont identiques ou différents et représentent un hydrogène ou un groupe alkyle C₁-C₆, à condition que G₃ ne représente pas un atome d'hydrogène lorsque L₃ représente une liaison directe ou du méthylène.

11. Composition pharmaceutique comprenant un dérivé de cyclosporine tel que défini dans la revendication 10 et un excipient, diluant ou support pharmaceutiquement acceptable.

12. Dérivé de cyclosporine tel que défini dans la revendication 10, destiné à être utilisé dans le traitement du corps humain ou animal.
